# EUROPÄISCHE PATENTSCHRIFT

(11) **EP 3 325 929 B1**
(45) Veröffentlichungstag und Bekanntmachung des Hinweises auf die Patenterteilung: **31.08.2022**
(21) Anmeldenummer: 16738355.3
(22) Anmeldetag: 01.07.2016
(51) Int. Cl.: G01J 5/07, G01J 5/00, G01J 5/53

(54) **TEMPERATURMESSEINRICHTUNG SOWIE WÄRMETHERAPIEVORRICHTUNG MIT EINER SOLCHEN MESSEINRICHTUNG**
TEMPERATURE MEASUREMENT DEVICE AND THERMAL THERAPY ARRANGEMENT WITH SUCH A MEASUREMENT DEVICE
DISPOSITIF DE MESURE DE TEMPÉRATURE ET ARRANGEMENT DE THÉRAPIE THERMIQUE AVEC LE DISPOSITIF DE MESURE DE TEMPÉRATURE

(30) Priorität: 17.07.2015 DE 102015009088
(43) Veröffentlichungstag der Anmeldung: 30.05.2018
(73) Patentinhaber: Drägerwerk AG & Co. KGaA, 23558 Lübeck (DE)
(72) Erfinder: FRANZ, Frank, 23617 Stockelsdorf (DE); KOCH, Jochim, 23909 Ratzeburg (DE); PILZ, Ulf, 23558 Lübeck (DE)
(86) Internationale Anmeldenummer: PCT/EP2016/001133
(87) Internationale Veröffentlichungsnummer: WO 2017/012697

(56) Entgegenhaltungen:
- EP-A1- 1 221 297
- DE-A1-102013 017 911
- ABBAS K ABBAS ET AL: "Neonatal non-contact respiratory monitoring based on real-time infrared thermography", BIOMEDICAL ENGINEERING ONLINE, BIOMED CENTRAL LTD, LONDON, GB, vol. 10, no. 1, 20 October 2011 (2011-10-20), page 93, XP021093710, ISSN: 1475-925X, DOI: 10.1186/1475-925X-10-93

## Beschreibung

Die Erfindung betrifft eine Messeinrichtung zur Bestimmung einer Temperatur eines Objekts, insbesondere einer Körpertemperatur eines Lebewesens, und ganz besonders einer Körpertemperatur eines Menschen gemäß Anspruch 1, eine Wärmetherapievorrichtung mit einer solchen Messeinrichtung nach Anspruch 9, die Verwendung einer solchen Messeinrichtung gemäß Anspruch 9, sowie zwei Verfahren, insbesondere zum Betrieb einer Messeinrichtung, nach den Ansprüchen 11 und 13.

Aus vielerlei Gründen ist die Bestimmung der Temperatur von Objekten erforderlich. Hierzu sind aus dem Stand der Technik eine Vielzahl an Messeinrichtungen und -sensoren bekannt.

Eine besondere Herausforderung stellt die Bestimmung der Temperatur eines Objektes dar, ohne dieses zu berühren. So wird beispielsweise in DE 10 2005 049 676 B3 beschrieben, wie berührungslos eine Oberflächentemperatur bzw. Kerntemperatur des Patienten mit Hilfe einer Infrarot-Messeinrichtung erfolgen kann. Die Bestimmung der Temperatur eines Babys in einem Inkubator mittels einer Infrarotkamera ist aus ABBAS K ABBAS ET AL: "Neonatal non-contact respiratory monitoring based on real-time infrared thermography",BIOMEDICAL ENGINEERING ONLINE, BIOMED CENTRAL LTD, LONDON, GB, Bd. 10, Nr. 1, 20. Oktober 2011 (2011-10-20), Seite 93, bekannt. Die Verwendung eines Kalibrierobjekts mit aktiver Heizung und Temperaturfühler ist aus DE 10 2013 017 911 A1 bekannt.

Problematisch ist, dass Infrarotkameras zur Aufnahme eines Patienten ungekühlt sind und deshalb eine geringe Messgenauigkeit aufweisen. Bei der medizinischen Körpertemperaturbestimmung besteht die Anforderung, dass 95% aller Messwerte innerhalb ± 0,5°C liegen, d.h. die Standardabweichung zwischen Messsystem und der Referenztemperatur muss kleiner als 0,25°C sein. Preiswerte Infrarotkameras haben jedoch eine Standardabweichung von >1°C, womit sie ungeeignet sind. Teure Infrarotkameras erreichen zwar Standardabweichung von circa 0,1 °C, können jedoch aus wirtschaftlichen Gründen oftmals nicht angeschafft werden.

### BESTATIGUNGSKOPIE

Aufgabe der Erfindung ist es daher, eine Messeinrichtung zur berührungslosen Bestimmung einer Temperatur eines Objekts, insbesondere einer Körpertemperatur eines Lebewesens, und ganz besonders einer Körpertemperatur eines Menschen, bereitzustellen, welche schnell arbeitet, zuverlässig eine hohe Messgenauigkeit erreicht und kostengünstig herstellbar ist.

Die vorstehende Aufgabe wird gelöst durch einen medizinischen Inkubator nach Anspruch 1 sowie durch ein Verfahren zur Erfassung der Oberflächentemperatur eines Babys nach Anspruch 8. Vorteilhafte Ausgestaltungen sind in den abhängigen Ansprüchen definiert.

Vorteilhaft hierbei ist, dass Messungenauigkeiten der Infrarotkamera mit Hilfe der permanent eingesetzten Kalibriervorrichtung ausgeglichen werden können. Bei der optischen Infrarotmessung eines Menschen ist der Emissionsgrad (auch Emissionskoeffizient genannt) der Haut zwar nicht exakt bekannt, man kann jedoch von einem Wert größer 0,9 bis 0,95 ausgehen (also ein "schwarzer Körper"). Wenn man gleichzeitig mit der Infrarotaufnahme des Patienten mit der Infrarotkamera die Kalibriervorrichtung im Fokus hat, kann eine aktuelle Kalibrierung bei jeder Temperaturbestimmung erfolgen. Damit ist es möglich, eine kostengünstige Messeinrichtung bereitzustellen, mit der die Temperatur eines Objekts, insbesondere eine Körpertemperatur eines Lebewesens, und ganz besonders eine Körpertemperatur eines Menschen, schnell, zuverlässig und mit hoher Messgenauigkeit bestimmbar ist

Mit dieser Methode lässt sich also die Schwäche in der Genauigkeit von preiswerten Infrarotkameras kompensieren und man erhält zu jeder Zeit eine kalibrierte Messung. Die Messgenauigkeit hängt dann im Wesentlichen von der Präzision des Temperatursensors ab. Selbstredend sollte die Außenhülle der Kalibriervorrichtung hierzu im Fokus der Infrarotkamera anordenbar sein. Optional ist die Außenhülle mit einer Haltevorrichtung mit der Infrarotkamera verbunden und mit der Haltevorrichtung im Fokus positioniert oder positionierbar. Vorzugsweise ist die Außenhülle im Randbereich des Fokus angeordnet. Durch eine feste Position im Bereich des Fokus ist es möglich, über eine Software den Bildbereich zu definieren, in welchem die Außenhülle liegt. Bei der optionalen Anwendung in einem medizinischen Inkubator kann die Haltevorrichtung beispielsweise an einer Haube des Inkubators ausgebildet sein. Die Infrarotkamera ist auch an der Haube festlegbar.

Gemäß einer Ausgestaltung der Messeinrichtung ist in der Außenhülle ein Heizelement angeordnet, das von einem Temperaturregler angesteuert ist, wobei der Temperatursensor eine Datenverbindung zum Temperaturregler aufweist. Damit ist es möglich, die Außenhülle auf die gleiche emittierte Strahlungsleistung der IR-Strahlung aufzuheizen wie die der zu messenden Oberflächenregion des Objektes. Eine mit einem Temperatursensor, zum Beispiel einem Widerstandsthermometer, bestimmte Temperatur in der Außenhülle entspricht dann der Oberflächentemperatur des Objektes.

In einer näheren Ausgestaltung der Erfindung, die insbesondere zur Bestimmung einer Körpertemperatur eines Menschen geeignet ist, ist vorgesehen, dass der außenseitige Emissionsgrad der Außenhülle ähnlich zum Emissionsgrad von menschlicher Haut ist, weiter bevorzugt zwischen 0,92 und 0,96 liegt und ganz besonders bevorzugt zwischen 0,935 und 0,945 liegt.

In einer speziellen Ausführungsform weist die Außenhülle außenseitig eine schwarze Außenfarbe (insbesondere ein Ofenlack) auf. Mit einer solchen kann ein definierter Emissionsgrad auf der Oberfläche bereitgestellt werden, der insbesondere im Bereich der menschlichen Haut liegt.

Weiterhin sieht eine nähere Ausführungsform der Messeinrichtung vor, dass die Außenhülle außenseitig einen Ofenlack oder eine eloxierte Schicht aufweist oder eine Oberflächenstruktur, die einen ähnlichen Emissionsgrad erzeugt. Mit diesen Herstellverfahren lässt sich eine Oberfläche mit homogenem Emissionsgrad bereitstellen.

In einer Ausführungsvariante der Messeinrichtung weist die Außenhülle ein Metallrohr auf. Die Rohrform ist geeignet dazu, den Temperatursensor und das Heizelement aufzunehmen. Die Materialwahl Metall erlaubt eine schnelle Temperaturänderung und somit Regelung auf eine Temperatur. Entsprechend schnell ist die Temperaturbestimmung des Objekts durchführbar. Vorzugsweise dient das Metallrohr als Trägerelement einer außenseitigen Beschichtung, insbesondere einer schwarzen Außenfarbe, einem Ofenlack, einer speziellen Oberflächenstruktur oder einer eloxierten Schicht.

Des Weiteren sieht eine nähere Ausgestaltung vor, dass die Infrarotkamera Infrarotstrahlung im Wellenlängenbereich von 8-14 µm detektiert, insbesondere die Strahlungsleistung in diesem Wellenlängenbereich. Diese Emissionswellenlängen im Spektralbereich von 8 bis 14 µm treten typischerweise in der Nähe der Umgebungstemperatur von Lebewesen auf. Man bezeichnet diesen Spektralbereich auch als mittleres und langwelliges Infrarot.

Eine hohe Präzision bei der Bestimmung der Temperatur wird bei einer Ausbildung erreicht, nach welcher der Temperatursensor ein NTC-Temperaturfühler (Negative Temperature Coefficient), ein PTC-Temperaturfühler (Positive Temperature Coefficient), ein Pt100-Temperaturfühler (Platin-Messwiderstände), ein Thermoelement oder eine Thermosäule aus Thermoelementen ist.

Platin-Messwiderstände werden nach ihrem Material und ihrem Nennwiderstand R₀ bei einer Temperatur von 0°C bezeichnet. Bei einem Pt100-Temperaturfühler beträgt der Nennwiderstand R₀ = 100 Ω.

Ein Thermoelement wandelt durch Thermoelektrizität Wärme in elektrische Energie um. Dazu werden zwei Leiter aus unterschiedlichen Materialien elektrisch miteinander verbunden. Zwischen ihren Enden liegt dabei eine Spannung an, die von der Temperaturdifferenz zwischen den Enden und der Kontaktstelle abhängt. Hintereinandergeschaltete Thermoelemente bilden eine Thermosäule.

Ergänzend sollte vorgesehen sein, dass die Außenhülle mit dem Temperaturfühler und/oder dem Heizelement thermisch gekoppelt ist. Hierdurch ändert sich die Temperatur der Oberfläche der Außenhülle besonders schnell. Die thermische Kopplung kann durch direkten Kontakt erfolgen, z.B. durch passgenaues Einsetzen des Temperaturfühlers und/oder dem Heizelement in die Außenhülle. Ergänzend oder optional können etwaige Zwischen- bzw. Hohlräume mit einem Fluid oder einer Paste gefüllt sein, zum Beispiel Wärmeleitpaste oder Wasser.

In einer bevorzugten Ausgestaltung der Messeinrichtung ist die Außenhülle dichter an der Infrarotkamera angeordnet als das Objekt. Damit liegt die Außenhülle nicht störend im Aktionsradius des Objekts bzw. Menschen.

Die Messeinrichtung ist besonders geeignet, wenn sie stationär aufgebaut ist, beispielsweise über einer Patientenliege oder im Bereich einer Personenkontrolle, z. B. auf Flughäfen und in Wärmetherapiegeräten (z.B. in medizinischen Inkubatoren, Wärmebetten, Wärmestrahlern). Es kann dann eine automatisierte Temperaturmessung durchgeführt werden.

Der Temperaturregelregler und eine etwaige Auswertevorrichtung können in einem oder mehrerer unabhängigen Gehäusen angeordnet sein. Sie können jedoch auch im Gehäuse der Kalibriervorrichtung oder im Gehäuse der Infrarotkamera integriert sein.

Des Weiteren betrifft die Vorrichtung eine Wärmetherapievorrichtung mit einer Personenaufnahme, insbesondere einer Liege oder einem Stuhl, und einer Messeinrichtung wie sie vorstehend beschrieben ist. Mit einer solchen Vorrichtung kann erfindungsgemäss ein medizinischer Inkubator bereitgestellt werden, mit dem die Oberflächentemperatur eines Babys kontinuierlich, automatisiert, kontaktlos und präzise gemessen sowie optional auch dokumentiert werden kann.

Die Erfindung betrifft außerdem die Verwendung eines vorstehend beschriebenen medizinischen Inkubators zur Bestimmung einer Oberflächentemperatur eines Babys Durch die Verwendung kann die Oberflächentemperatur eines Babys schnell, zuverlässig und mit hoher Messgenauigkeit bestimmt werden. Dabei ist eine kostengünstige Infrarotkamera einsetzbar, denn deren Messungenauigkeiten kann mit Hilfe der permanent eingesetzten Kalibriervorrichtung ausgeglichen werden.

In einer näheren Verwendungsform sind eine Messoberfläche des Objekts und die Außenhülle der Kalibriervorrichtung, insbesondere gleichzeitig, im Fokus der Infrarotkamera angeordnet.

Damit kann eine gleichzeitige Bestimmung der Strahlungsleistung der emittierten Wellenlängenbereiche der Außenhülle und der Messoberfläche erfolgen. Grundsätzlich wäre es jedoch auch möglich, die Strahlungsleistung der emittierten Wellenlängenbereiche der Außenhülle und der Messoberfläche nacheinander, d.h. zeitlich versetzt, zu bestimmen. Dies dauert in der Regel jedoch länger, ist unkomfortabler und kann zu Messungenauigkeiten führen.

Des Weiteren betrifft die Erfindung ein Verfahren nach Anspruch 8.

Die ausgegebene Temperatur entspricht dann der Oberflächentemperatur im Bereich der Messoberfläche des Objekts. Die Erfindung umfasst dabei auch Varianten, bei welchen die Temperatur in der Außenhülle über den gesamten Zeitraum angegeben wird und schließlich die Temperatur, bei welcher die Strahlungsleistung des emittierten Wellenlängenbereichs der Außenhülle gleich der Strahlungsleistung des emittierten Wellenlängenbereichs der Messoberfläche ist, visuell und/oder akustisch als Messergebnis gekennzeichnet wird, z. B. durch eine kontinuierliche statt blinkende Anzeige oder einen Piepton. Mit dem Verfahren ist es möglich, eine kostengünstige Messeinrichtung mit kostengünstiger Infrarotkamera bereitzustellen, mit der die Temperatur eines Objekts, insbesondere eine Körpertemperatur eines Lebewesens, und ganz besonders eine Körpertemperatur eines Menschen, schnell, zuverlässig und mit hoher Messgenauigkeit bestimmbar ist. Verfahrensgemäß ist die Messgenauigkeit nämlich im Wesentlichen von der Genauigkeit des Temperatursensors abhängig und nicht von der Genauigkeit der Infrarotkamera.

Das Regeln der Temperatur kann insbesondere durch ein Regeln der Strahlungsleistung des emittierten Wellenlängenbereichs der Außenhülle auf den Wert der Strahlungsleistung des emittierten Wellenlängenbereichs der Messoberfläche erfolgen, indem die Temperatur der Außenhülle verändert wird.

Eine nicht unter die Ansprüche fallende Alternative betrifft ein Verfahren, insbesondere zum Betrieb einer vorstehend beschriebenen Messeinrichtung, bei dem zunächst eine Messoberfläche eines Objekts und eine Außenhülle einer Kalibriervorrichtung im Fokus einer Infrarotkamera angeordnet werden. Danach erfolgt eine Bestimmung der Strahlungsleistung des emittierten Wellenlängenbereichs der Außenhülle und der Strahlungsleistung des emittierten Wellenlängenbereichs der Messoberfläche. Zudem wird die Temperatur der Außenhülle bestimmt. Danach wird ein Korrekturfaktor basierend auf dem Verhältnis zwischen der Temperatur der Außenhülle und der Strahlungsleistung des bestimmten emittierten Wellenlängenbereichs der Außenhülle ermittelt. Daraufhin erfolgt eine Zuordnung einer Temperatur zur bestimmten Strahlungsleistung des emittierten Wellenlängenbereichs der Messoberfläche unter Berücksichtigung des ermittelten Korrekturfaktors, welche dann ausgegeben wird.

Vorteilhaft an dem Verfahren ist, dass mit Hilfe des Korrekturfaktors, welcher immer aktuell ermittelt wird und die momentane Messabweichung beinhaltet, die am Objekt bestimmte Temperatur korrigiert wird. Geringfügigen Ungenauigkeiten durch unterschiedliche Emissionsgrad und der tatsächlichen Kerntemperaturen jeweils des Objekts und der Außenhülle, sind vernachlässigbar, insbesondere deutlich geringer als ohne Korrekturfaktor ermittelte Temperaturen des Objekts.

Gemäß einer optionalen Verfahrensergänzung erfolgt ein Regeln der Temperatur der Außenhülle auf einen definierten Wert, wobei das Ermitteln des Korrekturfaktors basierend auf dem Verhältnis zwischen dem definierten Wert und der Strahlungsleistung des bestimmten emittierten Wellenlängenbereichs der Außenhülle erfolgt.

Die Regelung auf eine definierte Temperatur hat den Vorteil, dass auch bei unbekannten oder veränderlichen Umgebungstemperaturen deutlich präzisere Temperarturmessungen möglich sind. Die Regelung kann bereits vor der eigentlichen Temperaturbestimmung gestartet werden. Die eigentliche Temperaturmessung des Objektes ist dann sehr schnell durchführbar, denn es treten kaum Trägheiten bei den Temperaturänderungen der Außenhülle auf, die abzuwarten wären.

Der definierte Wert, auf welchen die Temperatur in der Außenhülle optional geregelt wird, sollte vorzugsweise in etwa der erwarteten Temperatur des Objektes entsprechen. Bei einem Menschen beispielsweise zwischen 37°C und 39°C. Bei konstanten Umgebungsbedingungen, wie in Laboren oder anderen klimatisierten Räumen, ist die Regelung auch durch die externe Klimatisierung umsetzbar. Auch bei einer Annahme der dann vorliegenden Raumtemperatur durch die Außenhülle lässt sich bereits eine gute Kompensation von Messungenauigkeiten der Infrarotkamera erreichen. Bei inkonstanten Umgebungstemperaturen reicht für eine gute Kompensation auch eine Temperierung der Außenhülle auf eine Temperatur zwischen 25°C und 30°C.

Weitere Merkmale, Einzelheiten und Vorteile der Erfindung ergeben sich aus dem Wortlaut der Ansprüche sowie aus der folgenden Beschreibung eines Ausführungsbeispiels anhand der Zeichnung. Es zeigt:

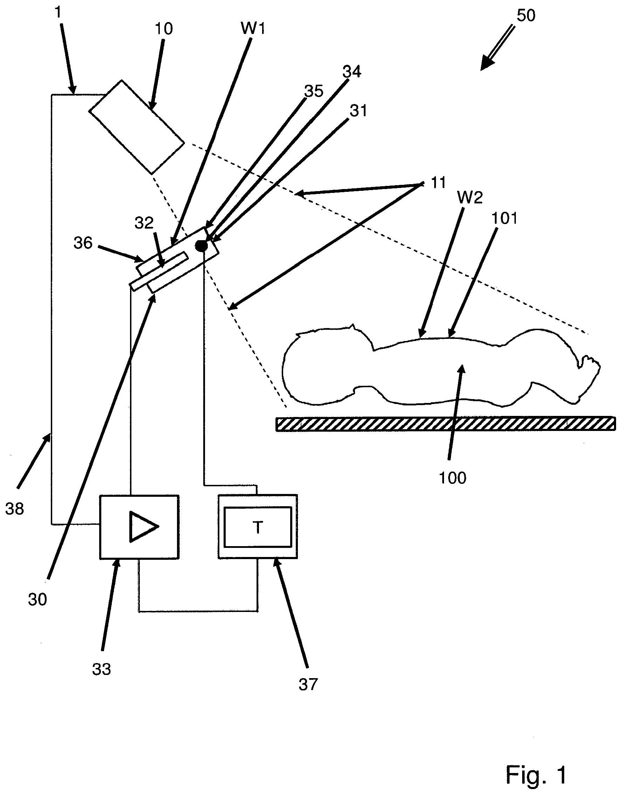

- Fig. 1: eine schematische Darstellung einer Wärmetherapievorrichtung aufweisend eines Messeinrichtung mit Infrarotkamera, in deren Fokus sich ein Objekt befindet.

Fig. 1 zeigt eine schematische Darstellung einer Wärmetherapievorrichtung 50 aufweisend eine Messeinrichtung 1 zur Bestimmung einer Temperatur T eines Objekts 100. Dabei handelt es sich vorliegend um einen Menschen, dessen Körpertemperatur bestimmt werden soll, nämlich um ein Baby in einem medizinischen Inkubator.

Die Messeinrichtung 1 weist eine Infrarotkamera 10 mit einem Fokus 11 auf, welche die Strahlungsleistung von Infrarotstrahlung im Wellenlängenbereich von 8-14 µm detektiert. In dem Fokus 11 sind das Objekt 100 und eine Außenhülle 31 einer Kalibriervorrichtung 30 angeordnet. Man erkennt, dass die Außenhülle 31 dichter an der Infrarotkamera 10 angeordnet ist als das Objekt 100. Dargestellt ist beispielhaft ein stationärer Aufbau der Messeinrichtung 1 über einer Patientenliege für Kleinkinder

Die Kalibriervorrichtung 30 ist über eine Datenverbindung 38 mit der Infrarotkamera 10 verbunden. Des Weiteren verfügt die Kalibriervorrichtung 30 über die Außenhülle 31, welche einen außenseitigen Emissionsgrad ähnlich dem Objekt 100 aufweist. Der außenseitige Emissionsgrad der Außenhülle 31 ist ähnlich zum Emissionsgrad von menschlicher Haut und liegt zwischen 0,92 und 0,96, bevorzugt zwischen 0,935 und 0,945. Dabei weist die Außenhülle 31 ein Metallrohr 35 auf, das außenseitig eine Beschichtung 36 trägt, die insbesondere eine schwarze Außenfarbe ist. Eine solche ist beispielsweise durch einen Ofenlack oder eine eloxierte Schicht ausbildbar.

In der Außenhülle 31 der Kalibriervorrichtung 30 ist ein Heizelement 32 angeordnet, das von einem Temperaturregler 33 angesteuert ist. Außerdem befindet sich in der Außenhülle 31 ein Temperatursensor 34, der eine Datenverbindung zum Temperaturregler 33 aufweist. Der Temperatursensor 34 ist zudem mit einem Anzeigemittel 37, insbesondere einem Display, zur Ausgabe der Temperatur T verbunden. Bei dem Temperatursensor 34 kann es sich um einen NTC-Temperaturfühler (Negative Temperature Coefficient), einen PTC-Temperaturfühler (Positive Temperature Coefficient), einen Pt100-Temperaturfühler (Platin-Messwiderstände), ein Thermoelement oder eine Thermosäule aus Thermoelementen handeln.

Eine solche Messeinrichtung 1 kann zur Bestimmung der Temperatur T des Objekts 100 eingesetzt werden. Hierzu sind eine Messoberfläche 101 des Objekts 100, die in Richtung der Infrarotkamera 10 zeigt, und die Außenhülle 31 der Kalibriervorrichtung 30 im Fokus 11 der Infrarotkamera 10 angeordnet. Die Messoberfläche emittiert einen Wellenlängenbereich W2 und die Außenhülle 31 einen Wellenlängenbereich W1.

Mit einer solchen Messeinrichtung ist nunmehr ein Verfahren durchführbar, bei dem folgende Schritte durchgeführt werden:
a) Anordnen der Messoberfläche 101 des Objekts 100 und der Außenhülle 31 der Kalibriervorrichtung 30 im Fokus 11 der Infrarotkamera 10;
b) Kontinuierliches Bestimmen der Strahlungsleistung des emittierten Wellenlängenbereichs W1 der Außenhülle 31 und der Strahlungsleistung des emittierten Wellenlängenbereichs W2 der Messoberfläche 101;
c) Regeln der Temperatur T in der Außenhülle 31 auf einen Wert, bei dem die Außenhülle 31 die gleiche Strahlungsleistung des Wellenlängenbereichs W1, W2 emittiert wie die Messoberfläche 101, dies insbesondere durch Regeln der Strahlungsleistung des emittierten Wellenlängenbereichs W1 der Außenhülle 31 auf den Wert der Strahlungsleistung des emittierten Wellenlängenbereichs W2 der Messoberfläche 101, indem die Temperatur T in der Außenhülle 31 verändert wird;
d) Ausgabe der Temperatur T, bei welcher die Strahlungsleistung des emittierten Wellenlängenbereichs W1 der Außenhülle 31 gleich der Strahlungsleistung des emittierten Wellenlängenbereichs W2 der Messoberfläche 101 ist.

Die ausgegebene Temperatur T entspricht dann der Kerntemperatur im Bereich der Messoberfläche 101 des Objekts 100.

Optional kann mit einer solchen Messeinrichtung auch ein Verfahren durchgeführt werden, bei dem folgende Schritte durchgeführt werden:
a) Anordnen der Messoberfläche 101 des Objekts 100 und der Außenhülle 31 der Kalibriervorrichtung 30 im Fokus 11 der Infrarotkamera 10;
b) Bestimmen der Strahlungsleistung des emittierten Wellenlängenbereichs W1 der Außenhülle 31 und der Strahlungsleistung des emittierten Wellenlängenbereichs W2 der Messoberfläche 101;
c) Bestimmen einer Temperatur der Außenhülle (31);
d) Ermitteln eines Korrekturfaktors basierend auf der bestimmten Strahlungsleistung des emittierten Wellenlängenbereichs W1 der Außenhülle 31 und Ermitteln des Korrekturfaktors basierend auf dem Verhältnis zwischen der Temperatur der Außenhülle (31) und der bestimmten Strahlungsleistung des emittierten Wellenlängenbereichs W1 der Außenhülle 31;
e) Zuordnen einer Temperatur T zu der bestimmten der Strahlungsleistung des emittierten Wellenlängenbereich W2 der Messoberfläche 101 unter Berücksichtigung des ermittelten Korrekturfaktors;
f) Ausgabe der Temperatur T.

Optional kann das Verfahren dadurch ergänzt sein, dass folgende Schritte durchgeführt werden:
- Regeln der Temperatur der Außenhülle (31) auf einen definierten Wert unter Schritt c);
- Ermitteln des Korrekturfaktors basierend auf dem Verhältnis zwischen dem definierten Wert und der Strahlungsleistung des bestimmten emittierten Wellenlängenbereichs (W1) der Außenhülle (31) unter Schritt d).

Die Erfindung ist nicht auf eine der vorbeschriebenen Ausführungsformen beschränkt, sondern in vielfältiger Weise abwandelbar.

### BEZUGSZEICHENLISTE

- 1: Messeinrichtung

- 10: Infrarotkamera
- 11: Fokus

- 30: Kalibriervorrichtung
- 31: Außenhülle
- 32: Heizelement
- 33: Temperaturregler
- 34: Temperatursensor
- 35: Metallrohr
- 36: Beschichtung
- 37: Anzeigemittel
- 38: Datenverbindung

- 50: Wärmetherapievorrichtung

- 100: Objekt
- 101: Messoberfläche

- T: Temperatur
- W1: emittierter Wellenlängenbereich (Außenhülle)
- W2: emittierter Wellenlängenbereich (Messoberfläche)

## Patentansprüche

1. Medizinischer Inkubator (50) mit einer Personenaufnahme und einer Messeinrichtung (1) zur Bestimmung einer Oberflächentemperatur eines Babys (100)
aufweisend eine Infrarotkamera (10) mit einem Fokus (11),
wobei eine Kalibriervorrichtung (30) über eine Datenverbindung mit der Infrarotkamera (10) verbunden ist,
wobei die Kalibriervorrichtung (30) eine Außenhülle (31) mit einem außenseitigen Emissionsgrad ähnlich dem Baby (100) aufweist,
wobei in der Außenhülle (31) ein Temperatursensor (34) angeordnet ist,
wobei in der Außenhülle (31) ein Heizelement (32) angeordnet ist, das von einem Temperaturregler (33) angesteuert ist,
wobei der Temperatursensor (34) eine Datenverbindung zum Temperaturregler (33) aufweist und
wobei die Messeinrichtung (1) zum Durchführen folgender Schritte ausgestaltet ist:
a) Regeln der Temperatur (T) in der Außenhülle (31) auf einen Wert, bei dem die Außenhülle (31) die gleiche Strahlungsleistung im Wellenlängenbereich (W1, W2) emittiert wie die Messoberfläche (101) des Babys (100);
b) Ausgabe der Temperatur (T), bei welcher die Strahlungsleistung des emittierten Wellenlängenbereichs (W1) der Außenhülle (31) gleich der Strahlungsleistung des emittierten Wellenlängenbereichs (W2) der Messoberfläche (101) ist.

2. Medizinischer Inkubator (50) nach Anspruch 1, wobei der außenseitige Emissionsgrad der Außenhülle (31) ähnlich zum Emissionsgrad von menschlicher Haut ist, weiter bevorzugt zwischen 0,92 und 0,96 liegt und ganz besonders bevorzugt zwischen 0,935 und 0,945 liegt.

3. Medizinischer Inkubator (50) nach einem der vorhergehenden Ansprüche, wobei die Außenhülle (31) außenseitig eine schwarze Außenfarbe aufweist.

4. Medizinischer Inkubator (50) nach einem der vorhergehenden Ansprüche, wobei die Außenhülle (31) außenseitig einen Ofenlack oder eine eloxierte Schicht aufweist.

5. Medizinischer Inkubator (50) nach einem der vorhergehenden Ansprüche, wobei die Außenhülle (31) ein Metallrohr (35) aufweist.

6. Medizinischer Inkubator (50) nach einem der vorhergehenden Ansprüche, wobei die Infrarotkamera (10) Infrarotstrahlung im Wellenlängenbereich von 8-14 µm detektiert.

7. Medizinischer Inkubator (50) nach einem der vorhergehenden Ansprüche, wobei der Temperatursensor (34) ein NTC-Temperaturfühler (Negative Temperature Coefficient), ein PTC-Temperaturfühler (Positive Temperature Coefficient), ein Pt100-Temperaturfühler (Platin-Messwiderstände), ein Thermoelement oder eine Thermosäule aus Thermoelementen ist.

8. Verfahren zur Erfassung einer Oberflächentemperatur eines Babys (100) in einem medizinischen Inkubator nach Anspruch 1, enthaltend die folgenden Schritte:
a) Anordnen einer Messoberfläche (101) des Babys (100) und einer Außenhülle (31) einer Kalibriervorrichtung (30) im Fokus (11) einer Infrarotkamera (10);
b) Bestimmen der Strahlungsleistung des emittierten Wellenlängenbereichs (W1) der Außenhülle (31) und des emittierten Wellenlängenbereichs (W2) der Messoberfläche (101);
c) Regeln der Temperatur (T) in der Außenhülle (31) auf einen Wert, bei dem die Außenhülle (31) die gleiche Strahlungsleistung im Wellenlängenbereich (W1, W2) emittiert wie die Messoberfläche (101);
d) Ausgabe der Temperatur (T), bei welcher die Strahlungsleistung des emittierten Wellenlängenbereichs (W1) der Außenhülle (31) gleich der Strahlungsleistung des emittierten Wellenlängenbereichs (W2) der Messoberfläche (101) ist.

## Claims

1. Medical incubator (50) with space for a person and a measuring device (1) for determining a surface temperature of a baby (100), comprising an infrared camera (10) with a focus (11),
a calibration apparatus (30) being connected to the infrared camera (10) via a data connection,
the calibration apparatus (30) having an outer shell (31) with an external emissivity similar to that of the baby (100),
a temperature sensor (34) being arranged within the outer shell (31), a heating element (32) that is driven by a temperature controller (33) being arranged in the outer shell (31),
the temperature sensor (34) having a data connection to the temperature controller (33) and
the measuring device (1) being configured to carry out the following steps:
a) adjusting the temperature (T) in the outer shell (31) to a value at which the outer shell (31) emits the same radiant flux in the wavelength range (W1, W2) as the measurement surface (101) of the baby (100);
b) outputting the temperature (T) at which the radiant flux of the emitted wavelength range (W1) of the outer shell (31) equals the radiant flux of the emitted wavelength range (W2) of the measurement surface (101).

2. Medical incubator (50) according to Claim 1, wherein the external emissivity of the outer shell (31) is similar to the emissivity of human skin, more preferably ranges between 0.92 and 0.96 and very particularly preferably ranges between 0.935 and 0.945.

3. Medical incubator (50) according to either of the preceding claims, wherein the outer shell (31) externally has a black external colour.

4. Medical incubator (50) according to any one of the preceding claims, wherein the outer shell (31) externally has a stoving enamel or an anodized layer.

5. Medical incubator (50) according to any one of the preceding claims, wherein the outer shell (31) has a metal pipe (35).

6. Medical incubator (50) according to any one of the preceding claims, wherein the infrared camera (10) detects infrared radiation in the wavelength range of 8-14 µm.

7. Medical incubator (50) according to any one of the preceding claims, wherein the temperature sensor (34) is an NTC (negative temperature coefficient) temperature probe, a PTC (positive temperature coefficient) temperature probe, a Pt100 (platinum measuring resistors) temperature probe, a thermocouple or a thermopile made of thermocouples.

8. Method for measuring a surface temperature of a baby (100) in a medical incubator according to Claim 1, containing the following steps:
a) arranging a measurement surface (101) of the baby (100) and an outer shell (31) of a calibration apparatus (30) in the focus (11) of an infrared camera (10);
b) determining the radiant flux of the emitted wavelength range (W1) of the outer shell (31) and of the emitted wavelength range (W2) of the measurement surface (101);
c) adjusting the temperature (T) in the outer shell (31) to a value at which the outer shell (31) emits the same radiant flux in the wavelength range (W1, W2) as the measurement surface (101);
d) outputting the temperature (T) at which the radiant flux of the emitted wavelength range (W1) of the outer shell (31) equals the radiant flux of the emitted wavelength range (W2) of the measurement surface (101).

## Revendications

1. Incubateur médical (50) comprenant un logement de personne et un dispositif de mesure (1) servant à la détermination d'une température de surface d'un nourrisson (100),
possédant une caméra infrarouge (10) ayant un foyer (11),
un arrangement d'étalonnage (30) étant relié à la caméra infrarouge (10) par le biais d'une liaison de données,
l'arrangement d'étalonnage (30) possédant une enveloppe externe (31) avec un degré d'émission côté extérieur similaire au nourrisson (100),
un capteur de température (34) étant disposé dans l'enveloppe externe (31), un élément chauffant (32) étant disposé dans l'enveloppe externe (31), lequel est commandé par un régulateur de température (33),
le capteur de température (34) possédant une liaison de données avec le régulateur de température (33) et
le dispositif de mesure (1) étant configuré pour exécuter les étapes suivantes :
a) régulation de la température (T) dans l'enveloppe externe (31) à une valeur à laquelle l'enveloppe externe (31) émet la même puissance de rayonnement dans la plage de longueurs d'onde (W1, W2) que la surface de mesure (101) du nourrisson (100) ;
b) délivrance en sortie de la température (T) à laquelle la puissance de rayonnement de la plage de longueurs d'onde (W1) émise de l'enveloppe externe (31) est égale à la puissance de rayonnement de la plage de longueurs d'onde (W2) émise de la surface de mesure (101).

2. Incubateur médical (50) selon la revendication 1, le degré d'émission côté extérieur de l'enveloppe externe (31) étant similaire au degré d'émission de la peau humaine, de préférence compris entre 0,92 et 0,96 et plus particulièrement de préférence compris entre 0,935 et 0,945.

3. Incubateur médical (50) selon l'une des revendications précédentes, l'enveloppe externe (31) possédant une couleur extérieure noire du côté extérieur.

4. Incubateur médical (50) selon l'une des revendications précédentes, l'enveloppe externe (31) possédant un vernis au four ou une couche anodisée du côté extérieur.

5. Incubateur médical (50) selon l'une des revendications précédentes, l'enveloppe externe (31) possédant un tube métallique (35).

6. Incubateur médical (50) selon l'une des revendications précédentes, la caméra infrarouge (10) détectant le rayonnement infrarouge dans la plage de longueurs d'onde de 8 à 14 µm.

7. Incubateur médical (50) selon l'une des revendications précédentes, le capteur de température (34) étant une sonde de température à CTN (coefficient de température négatif), une sonde de température à CTP (coefficient de température positif), une sonde de température Pt100 (résistance de mesure au platine) ou un thermocouple ou encore une thermopile composée de thermocouples.

8. Procédé d'acquisition d'une température de surface d'un nourrisson (100) dans un incubateur médical selon la revendication 1, comprenant les étapes suivantes :
a) disposition d'une surface de mesure (101) du nourrisson (100) et d'une enveloppe externe (31) d'un arrangement d'étalonnage (30) dans le foyer (11) d'une caméra infrarouge (10) ;
b) détermination de la puissance de rayonnement de la plage de longueurs d'onde (W1) émise de l'enveloppe externe (31) et de la plage de longueurs d'onde (W2) émise de la surface de mesure (101) ;
c) régulation de la température (T) dans l'enveloppe externe (31) à une valeur à laquelle l'enveloppe externe (31) émet la même puissance de rayonnement dans la plage de longueurs d'onde (W1, W2) que la surface de mesure (101) ;
d) délivrance en sortie de la température (T) à laquelle la puissance de rayonnement de la plage de longueurs d'onde (W1) émise de l'enveloppe externe (31) est égale à la puissance de rayonnement de la plage de longueurs d'onde (W2) émise de la surface de mesure (101).
